(19) **Europäisches Patentamt / European Patent Office / Office européen des brevets**

(11) **EP 3 472 341 B1**

(12) **EUROPEAN PATENT SPECIFICATION**

(45) Date of publication and mention
of the grant of the patent:
**15.04.2026  Bulletin 2026/16**

(21) Application number: **17737724.9**

(22) Date of filing: **16.06.2017**

(51) International Patent Classification (IPC):
*C12Q 1/44* (2006.01)     *G01K 17/04* (2006.01)
*G01N 33/92* (2006.01)

(52) Cooperative Patent Classification (CPC):
**C12Q 1/44; G01N 33/92**

(86) International application number:
**PCT/EP2017/064850**

(87) International publication number:
**WO 2017/216381 (21.12.2017 Gazette 2017/51)**

(54) **METHOD FOR CALORIMETRIC DETERMINATION OF THE LIPOPROTEIN LIPASE ACTIVITY IN HUMAN PLASMA ENVIRONMENT**

VERFAHREN ZUR KALORIMETRISCHEN BESTIMMUNG DER LIPOPROTEINLIPASEAKTIVITÄT IN MENSCHLICHEM PLASMA

PROCÉDÉ DE DÉTERMINATION CALORIMÉTRIQUE DE L'ACTIVITÉ LIPOPROTÉINE LIPASE DANS UN ENVIRONNEMENT DE PLASMA HUMAIN

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: **16.06.2016  US 201662350747 P**

(43) Date of publication of application:
**24.04.2019  Bulletin 2019/17**

(73) Proprietor: **Tallinn University of Technology**
**19086 Tallinn (EE)**

(72) Inventors:
• **LÕOKENE, Aivar**
  **19086 Tallinn (EE)**
• **REIMUND, Mart**
  **19086 Tallinn (EE)**
• **KOVROV, Oleg**
  **SE-901 87 Umea (SE)**
• **OLIVERCONA, Gunilla**
  **SE-901 87 Umea (SE)**

(74) Representative: **Sarap, Margus**
**Jalaka 42-23**
**50109 Tartu (EE)**

(56) References cited:
**US-A1- 2012 064 556**

• WU XULI ET AL: "Acteoside: A lipase inhibitor from the Chinese teaLigustrum purpurascenskudingcha", FOOD CHEMISTRY, vol. 142, 25 July 2013 (2013-07-25), pages 306 - 310, XP028706674, ISSN: 0308-8146, DOI: 10.1016/J.FOODCHEM.2013.07.071
• REIMUND MART ET AL: "Lipoprotein lipase activity and interactions studied in human plasma by isothermal titration calorimetry", JOURNAL OF LIPID RESEARCH,, vol. 58, no. 1, 31 December 2016 (2016-12-31), pages 279 - 288, XP009500556, ISSN: 1539-7262
• HANSEN LEE D ET AL: "Enzyme-catalyzed and binding reaction kinetics determined by titration calorimetry", BIOCHIMICA ET BIOPHYSICA ACTA (BBA) - GENERAL SUBJECTS, ELSEVIER, AMSTERDAM, NL, vol. 1860, no. 5, 22 December 2015 (2015-12-22), pages 957 - 966, XP029449726, ISSN: 0304-4165, DOI: 10.1016/J.BBAGEN.2015.12.018

## Description

## Field of the invention

The present invention relates to the field of calorimetric drug discovery, specifically to a method for calorimetric determination of the lipoprotein lipase activity in human plasma environment performing isothermal titration calorimetry measurements for measuring LPL activity in plasma.

## Background of the invention

The hydrolytic breakdown of plasma triglycerides by lipoprotein lipase (LPL) at the vascular endothelium is a crucial factor that determines the triglyceride level in plasma (Olivecrona 2016). Numerous recent studies suggest that elevated levels of triglyceride-rich lipoproteins in plasma are an independent risk factor of atherosclerosis (Nordestgaard 2016; Musunuru & Kathiresan 2016; Khetarpal & Rader 2015). Therefore, LPL has regarded as a potential drug target in lowering triglyceride levels (Rader 2016; Dallinga-Thie et al. 2016). The rate of lipolysis does not only depend on the production of LPL - several plasma components have been shown to directly or indirectly modulate the activity or stability of LPL. Apolipoproteins C-II (apoC-II) and apolipoprotein A-V (apoA-V) increase the rate of LPL while apolipoproteins C-I, C-III (apoC-I, apoC-III) and angiopoietin-like proteins 3, 4, 8 (ANGPTL 3, 4, 8) behave as inhibitors of the lipolysis (Olivecrona 2016; Dijk & Kersten 2016). Due to polymorphisms and posttranslational modifications, LPL and its regulator proteins can exist in multiple forms. Expression of these proteins depends on physiological status, such as fasting/feeding, exercises or temperature (Olivecrona 2016; Dijk & Kersten 2016). Apart from the substrate lipoproteins, triglyceride-rich VLDL and chylomicrons, LPL binds cholesterol-rich LDL with appreciable affinity (Lookene et al. 1997). Both genetic and environmental factors influence concentration and proportion of these lipoproteins. Free fatty acids, products of the lipolysis, can affect LPL activity directly or via its ligands (Robal et al. 2012; Peterson et al. 1990). Thus, it is reasonable to consider that the efficiency of the hydrolytic degradation of triglycerides by LPL depends on coaction of numerous different extracellular compounds whose concentrations can vary. Moreover, since plasma is a complex fluid containing thousands of proteins, and other components, the majority of them are present in very low concentrations, it is possible that some plasma regulators of LPL have not been identified yet. Due to the crowding effect, even macromolecular environment of plasma itself may influence LPL interactions with its ligands. The protein concentration of plasma 80 g/liter has been shown to be sufficient to cause significant crowding effect (Ellis 2001). There is another aspect that is specific and important for lipases - these enzymes act at the surface of aggregated substrates that is continuously changing during the lipolysis, resulting to complex reaction kinetics (Eisenberg & Olivecrona 1979). It is likely that the influence of the ligands on LPL can change during the lipolysis.

Lack of a suitable technique for continuous monitoring of lipolysis in blood plasma has hampered the understanding of the action of LPL under physiological conditions. Typically, LPL activity is measured using artificial systems of radiolabeled (Briquet-Laugier et al. 1999), fluorogenic (Basu et al. 2011) or chromogenic (Panteghini et al. 2001) substrates, and the reaction products are detected in stopped assays by chemical quantification or by determination of radioactivity. As a rule, only the initial rates are determined in these assays. Those studies have provided essential information about several aspects of the LPL action. For example, determination of LPL activity in post-heparin plasma, using artificial substrate systems, is used for estimation of amount of active LPL molecules at the vascular endothelium (Tornvall et al. 1995). However, those measurements are not sufficient to entirely evaluate the rate of lipolysis in plasma. First, the activity of LPL on non-physiological substrates can be less affected by its ligands (Olivecrona & Beisiegel 1997) and is more resistant to thermal inactivation (Lookene et al. 2004) or proteolytic cleavage (Lookene & Bengtsson-Olivecrona 1993) than its activity on lipoproteins. Second, as discussed above, the activity of LPL in vivo is influenced by numerous plasma components (Olivecrona 2016). In summary, determination of LPL activity in postheparin plasma using artificial substrate systems is likely not to be sufficient for estimation of the real lipolysis rate in vivo.

US 2012/064556A1, JIN WEIJUN, 15.03.2012 discloses a method for the measurement of LPL. The method comprises the steps of administering a dosage of an anti-coagulant to the subject, gathering the biological sample, adding the biological sample to a well of a well plate, adding a first buffer to the well plate, adding a second buffer to the well plate, adding a substrate to the well plate, incubating the well plate, measuring the fluorescence of the material within the well plate, and correlating the measured fluorescence to a level of LPL activity.

WU XULI ET AL: "Acteoside: A lipase inhibitor from the Chinese tea Ligustrum purpurascenskudingcha", FOOD CHEMISTRY, vol. 142, 25 July 2013, pages 306-3 discloses the use of ITC to the study of interactions between acteoside and a lipase characterizing acteoside and lipase by fluorescence spectroscopy. The isothermal titration calorimetry and circular dichroism revealing that acteoside might act as a non-competitive lipase inhibitor.

REIMUND MART ET AL: "Lipoprotein lipase activity and interactions studied in human plasma by isothermal titration calorimetry", JOURNAL OF LIPID RESEARCH,, vol. 58, no. 1, 31 December 2016 (2016-12-31), pages 279-288 is disclosed the use of ITC for the study of enzymatic reactions and emphasizes the advantages of ITC in that complex solutions, such as opaque and multi-phase solutions as well.

Thus, the problem of known methods is that there is no sufficient method to evaluate the rate of lipolysis and measure lipoprotein lipase activity in human blood plasma to determine risk factors of atherosclerosis, to test how added regulators influence the lipoprotein lipase reaction in plasma, to find out the causes of hypertriglyceridemia.

## Brief description of the invention

The aim of the method for calorimetric determination of the lipoprotein lipase activity in human plasma environment according to present invention is to provide an effective method and tool for finding out causes of hypertriglyceridemia and assay for testing potential drug candidates against hypertriglyceridemia under in-vivo like conditions, to evaluate the rate of lipolysis and measure lipoprotein lipase activity in human blood plasma to determine risk factors of atherosclerosis, to test how added regulators influence the lipoprotein lipase reaction in plasma.

The aim is achieved by isothermal titration calorimetry. Lipoprotein lipase (LPL) is a key enzyme in the hydrolytic degradation of triglyceride-rich lipoproteins in blood plasma. Activity and stability of LPL is regulated by numerous plasma components (Olivecrona 2016). Previous studies performed under artificial conditions have provided essential information about several aspects of the LPL action. However, the prior art of how the ligands affect LPL under native conditions that is plasma environment is still limited.

The present invention demonstrates that isothermal titration calorimetry (ITC) according to the present invention overcomes several limits of prior art techniques. First, ITC measurements can be successfully performed in minimally diluted human plasma. Second, the ITC based assay of LPL is highly sensitive, ITC can be used for determination of LPL activity in postheparin plasma. The estimated detection limit for LPL is as low as 50 pM. Third, ITC enables quantitative comparison of the properties of plasma samples from patients and control subjects as substrates for LPL. This can be done by recording kinetics of total substrate hydrolysis by LPL. Fourth, ITC provides new and detailed information about how ligands influence LPL in plasma. Using ITC, it is demonstrated that 1) increasing of apoC-III concentration in plasma reduces LPL activity on triglyceride rich lipoproteins but not on triglyceride poor fraction of lipoproteins 2) ANGPTL3 and 4 suppress the activity of LPL in plasma via analogous mechanism- in both cases a fast phase inhibition and a slow phase inactivation can be clearly distinguished.

The method according to the present inventions demonstrates that isothermal titration calorimetry (ITC) overcomes several limits of other techniques by providing a continuous assay using the observable thermal power that is directly proportional to the rate of the lipolysis. Raw data of ITC experiments are presented as thermograms in which the changes in the heat rate (heat flow, thermal power, heat flux) are monitored at constant temperature (Bianconi 2005). ITC can be used for determination of LPL activity in plasma, for registration of total lipolysis kinetics or for investigations of ligand effects on LPL in plasma. Considering that increasing of LPL activity through blocking inhibitory effect of its ligands is one possible way for lowering plasma triglyceride levels (Rader 2016; Dallinga-Thie et al. 2016), the ITC-based approaches proposed in this report can be used for testing of drug candidates in human plasma.

The method according to the present invention does not need chromogenic, fluorogenic or radioisotope labelled ligands that are used in known enzyme assay methods and is used to obtain data that are not attainable by prior art techniques.

The method according to the present invention provides reliable and reproducible data about the action of LPL in plasma that is the physiologic environment for this enzyme. The advantage of ITC stems from possibility to perform measurements directly in plasma, using observable changes in heat flow for determination of reaction rate. However, in the examination of the ITC experiments of plasma lipolysis, it is important to take into account that determined heat rate is a complex parameter which includes heat effects of four different processes: hydrolysis of ester bonds, structural changes of lipoproteins, fatty acid neutralization by the buffer and binding of fatty acids to albumin. Several observations of this study suggest that the heat rate is a suitable quantitative parameter for measurement of catalytic activity of LPL in plasma. First, LPL when added to plasma was stable in the ITC cell for a long time period. Second, the heat produced by the action of LPL was proportional to the amount of released fatty acids. Third, there was a linear dependence between LPL concentration and heat flow. Fourth, in the case of complete hydrolysis, the total heat production was linearly dependent on the initial triglyceride concentration. Fifth, the heat effect was nearly the same regardless of whether used substrate for LPL was synthetic triglyceride emulsion Intralipid, VLDL or triglyceride rich plasma. Based on this, it is reasonable to assume that the heat effect accompanying with structural changes of lipoproteins or due to other interactions established in the system are not significant in comparison with those due to hydrolysis of ester bonds. In principle, activity of LPL in plasma can be measured by detecting produced fatty acid after stopping the reaction. However, since plasma contains fatty acids at high concentrations (0.3 - 1 mM) (Dole 1956), these assays are applicable only when significant amount of fatty acids have been produced by added LPL. Thus, detection of produced fatty acids has limited usage in investigation of LPL action in plasma. In addition, these measurements do not provide continuous data and are more laborious and time consuming than the ITC measurements.

The ITC assay for measurement of initial rates was sufficiently sensitive to determine LPL activity in postheparin plasma. Thus, the ITC assay is a promising alternative to assays that use radiolabeled or fluorogenic substrates or measure released fatty acids by NEFA kit. The advantage of ITC - no additional reagents are needed to perform the experiments and minimally diluted human plasma can be used. The possibility to use plasma as a source of triglycerides is also advantage, since both catalytic activity and ligand interactions of LPL are dependent on the used substrate system (Lookene et al. 2004; Olivecrona & Beisiegel 1997). In humans, LPL and hepatic lipase (HL)are bound to the vascular endothelium, and their activities have been analyzed in plasma obtained from blood samples collected after intravenous injection of heparin, a procedure to release LPL and HL from cellular proteoglycans (Tornvall et al. 1995). By using inhibitory antibodies of HL the detected activity in post-heparin plasma can be almost completely attributed to LPL. An ITC based assay for determination of LPL activity in plasma using commercially available VLDL or Intralipid is devised for general use.

In addition to determination of initial rate of hydrolysis, ITC allows continuous monitoring of total lipolysis in plasma. This is important since the effect of different plasma components on LPL may depend on the concentration of triglycerides or composition of the lipoprotein fraction. Determination of the initial activity might not be sufficient to evaluate how efficiently triglycerides are hydrolysed by LPL. Transformation of a single lipolysis time course into the plot of heat rate versus remaining substrateconcentration (hydrolysable ester bonds) revealed how the reaction rate was changing during degradation of triglyceride rich lipoproteins to remnant particles. This kind of analysis gives proper evaluation for ability of LPL to degrade lipoproteins of a certain plasma sample. Although triglyceride concentrations vary between people, recordings of total hydrolysis curves make lipolysis comparable. The comparison of total hydrolysis of two randomly selected plasma samples clearly demonstrated that there are individual differences in efficiency of lipolysis of plasma lipoproteins. Thus, the analysis of total hydrolysis curves can be used to ascertain whether hypertriglyceridemia is caused by plasma composition.

Continuous monitoring of the LPL catalyzed reactions provided detailed information about how apoC-III, ANGPTL3 and ANGPTL4 suppress LPL activity in plasma. These proteins are regarded as potential targets for drug development with the aim to lower triglyceride levels in plasma (Rader 2016; Dallinga-Thie et al. 2016).

It is preferred to investigate their inhibitory mechanism in human plasma. ApoC-III could only moderately affect LPL activity in plasma - increasing its concentration to 50 $\mu$M reduced the activity of LPL about by 30%. This is different from the data obtained by isolated chylomicrons - in that case complete inhibition was ob-

served already at 10 $\mu$M apoC-III (Larsson et al. 2013). Thus, apoC-III was less potent LPL inhibitor in plasma than in an assay of isolated chylomicrons. Assuming that apoC-III displaces LPL from lipoproteins (Larsson et al. 2013), it is reasonable to propose that plasma contains components that reduce apoC-III ability to inhibit binding of LPL to triglyceride rich lipoproteins. Analysis of the total hydrolysis curves demonstrated that apoC-III affected LPL activity on triglyceride rich lipoproteins but not on triglyceride poor fraction of lipoproteins. This can be explained by leaving of apoC-III from the surface of lipoproteins when their triglyceride content is decreasing. Effect of ANGPTL4and ANGPTL3 on LPL in plasma was detectable already at nanomolar concentrations. However, these concentrations were at least one magnitude higher than the estimated plasma concentrations of these proteins (Robciuc et al. 2010). Thus, real plasma concentrations of these proteins are too low to affect activity of LPL. Although ANGPTL4was more efficient in lowering of LPL activity thanANGPTL3, the inhibition time courses of the two proteins were similar - in both cases it was possible to clearly distinguish a fastand a slow phase. This observation suggest analogous inhibition mechanisms. There is no consensus by which mechanisms ANGPTL3 and 4 suppress the activity of LPL. It has been proposed that ANGPTL3and ANGPTL4 inhibit LPL through different mechanisms (Shan et al. 2009) and that only ANGPTL4induces irreversible inactivation of LPL (Sukonina et al. 2006). However, it is also proposed that ANGPTL4acts as a non-competitive inhibitor LPL, forming a reversible complex with LPL (Lafferty et al. 2013). It is important to stress that these two mechanisms are not mutually exclusive - reversible complex formation can precede to the inactivation. It is tempting to speculate that both mechanisms exist in plasma. Increasing of LPL activity via reducing the inhibitory effect of apoC-III, ANGPTL3 or ANGPTL4 is one possible way to lower triglyceride levels (Rader 2016; Dallinga-Thie et al. 2016). ITC allows testing of possible drug candidates that block the action of these proteins in human plasma.

The invention is set out in the appended set of claims.

## Brief description of the drawings

The preferred embodiment of present invention is explained more precisely with following figures added, where

Figures 1a to Figure 1d show enzymatic activity of LPL as measured by ITC.

Figure 1a illustrates the schematic model of the ITC system used in the present method, wherein the ITC cell is filled with human plasma, LPL is injected to the ITC cell from the syringe-stirrer.

Figure 1b shows the heat rate change as a result of a single injection of LPL into ITC cell with 1 ml of human plasma. TG and LPL concentration in the ITC cell is for example 2.7 mM and 500 pM, respectively. The change of heat rate dQ/dt is proportional to the reaction rate v.

Figure 1c shows sequential injections of LPL into the same aliquot of plasma as in figure 1b. Each injection increased LPL concentration in the ITC cell by 230 pM.

Figure 1d illustrates the data obtained from figure 1c presented as relationship of heat rate versus LPL concentration.

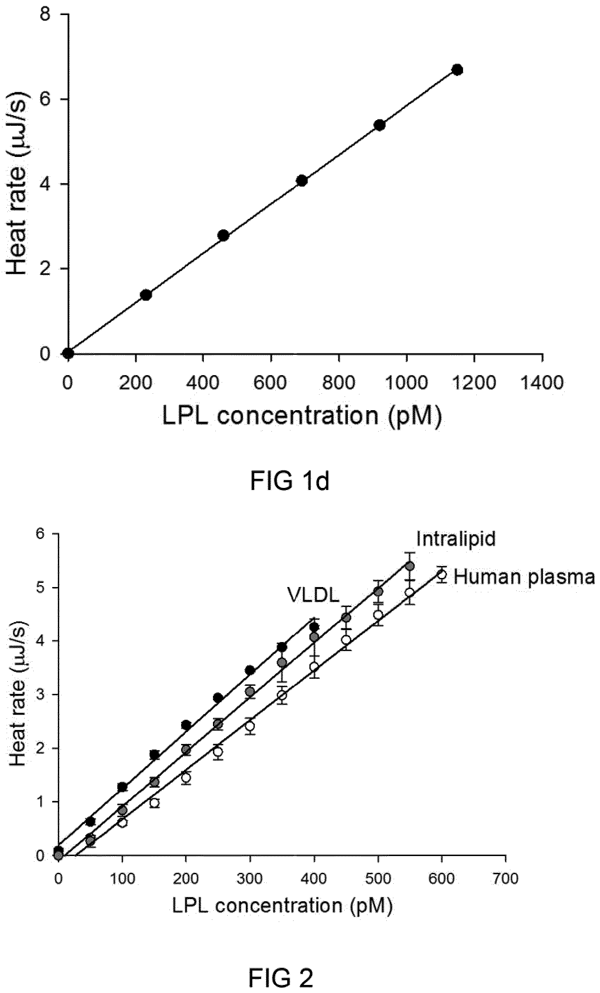

Figure 2. illustrates ITC-based assays for determination of LPL activity using different substrate systems. Sequential injections of LPL into human plasma, substrate mixture containing human VLDL or in alternative embodiment of present invention substrate mixture containing Intralipid. In all measurements, triglyceride concentration was 1 mM and each injection increased LPL concentration by 50 pM. The slopes of the lines were as follows: human plasma 0.0092, Intralipid 0.0102, and VLDL 0.0106. Human plasma contained 20 mM TRIS, pH 7.4. VLDL substrate mixture contained 10 mg/ml BSA, 10 IE/ml heparin, 20 mM TRIS pH 7.4, 0.15 M NaCl. Intralipid substrate mixture contained 10 mg/ml BSA, 10 IE/ml heparin, 63 % goat inactivated serum (source for apoC-II), 20 mM TRIS pH 7.4, 0.15 M NaCl. The values are mean ± S.D. of three different measurements.

Figure 3a and figure 3b illustrate post-heparin plasma LPL activity as measured by ITC, said experiment involving injection of post-heparin plasma is not part of the invention.

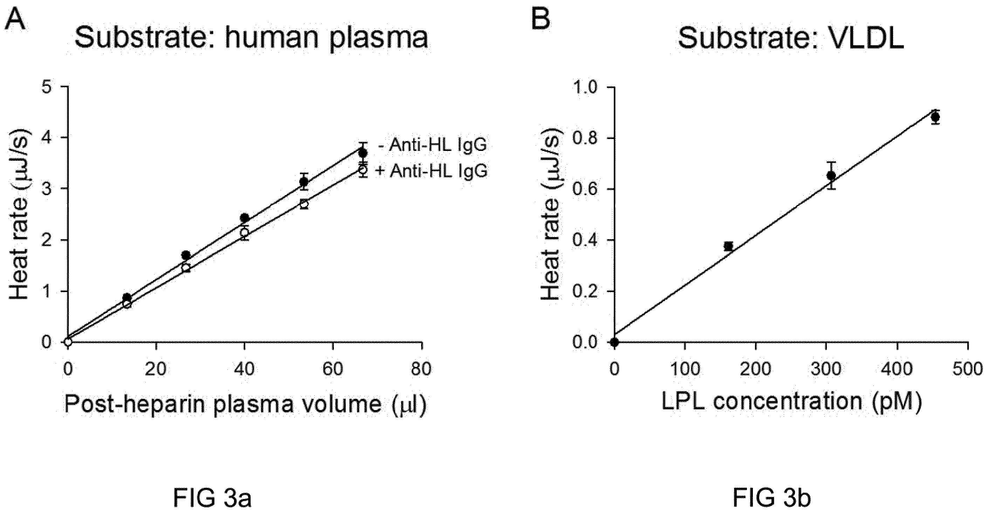

Figure 3a illustrates the activity, wherein five sequential injections of human postheparin plasma (1.5 times diluted) with (+Anti-HL IgG) or in alternative embodiment of present invention without (- Anti-HL IgG) hepatic lipase inhibitor (antibodies to HL) were made into 1 ml of human pre-heparin plasma. Final concentration of triglycerides of pre-heparin plasma was 1 mM. Antibodies to HL were added to the post-heparin plasma to evaluate the contribution of hepatic lipase on the total activity. Concentration of anti-HL IgG was 1.5 mg/ml.

Figure 3b illustrates the heat rate, wherein three sequential injections of 20 μl of human post-heparin plasma were made into 1 ml of substrate mixture containing VLDL (0.42 mM triclyceride and 0.1 mg/ml protein), 10 mg/ml BSA, 10 IE/ml heparin in 10 mM TRIS, 0.15 M NaCl, pH 7.4 buffer. The concentration of LPL in the postheparin plasma was 7.3 nM based on Figures 3A and 2, human plasma. The values are mean ± S.D. of three different determinations.

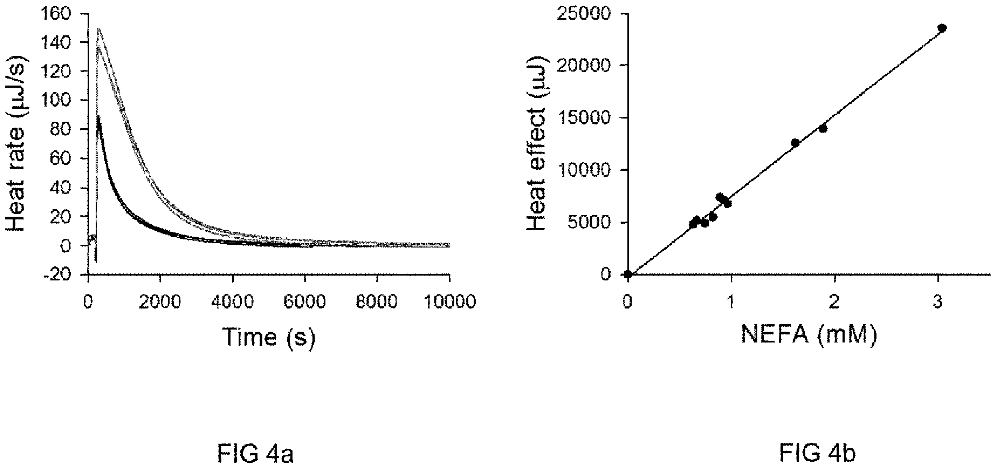

Figure 4a to figure 4c illustrate total hydrolysis of human plasma lipids by LPL as measured by ITC.

Figure 4a illustrates raw thermograms of total hydrolysis of human plasma lipids by LPL. Experiments were performed with plasma sample that contained 1 mM or 2.7 mM triglycerides. Concentration of LPL was 22 nM. Three measurements with both plasma samples are presented.

Figure 4b shows relationship between total heat production and concentration of released fatty acids. Heat rates were obtained from full hydrolysis of 10 different human plasma samples on MicroCal Auto-ITC200. In these experiments 200 μl of plasma was hydrolyzed by 8 nM LPL for one hour. The surface areas of the curves for full hydrolysis were calculated in the MicroCal Origin and compared with the release of free fatty acids (final concentration minus initial concentration) measured using NEFA kit.

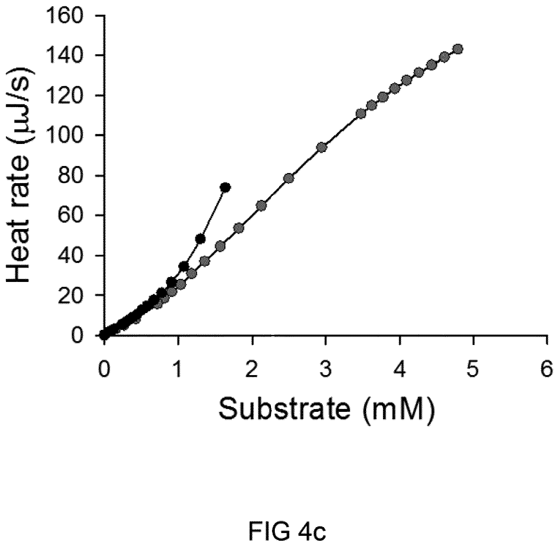

Figure 4c presents transformation of total hydrolysis data presented in figure 4a into relationship between heat rate and remaining substrate concentration (expressed as concentration of hydrolysable ester bonds).

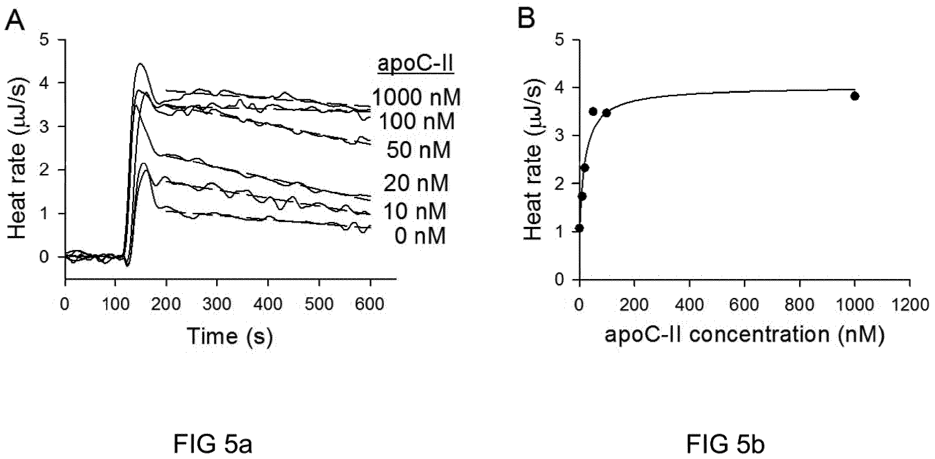

Figure 5a and figure 5b illustrate Activation of LPL by apoC-II.

Figure 5a shows raw ITC thermograms of Intralipid hydrolysis by LPL in the absence or in alternative embodiment of present invention presence of different concentrations of apoC-II. The substrate mixture composition was as follows: 1 mg triglyceride/ml Intralipid, 10 mg/ml BSA, 10 IU/ml heparin, 0.15 M NaCl, 10 mM TRIS pH 7.4. Single injection of LPL were made into the substrate mixture that contained or in alternative embodiment of present invention did not contained apoC-II. Final concentration of LPL was 1.2 nM in all experiments.

Figure 5b shows hydrolysis heat rate as a function of apoC-II concentration. Heat rate points at 200 s were obtained from figure 5a. The data were fitted using the following equation (Quinn et al. 1983):

$$v = k_{cat} \cdot L \cdot \left( \frac{\beta \cdot C + K_d}{C + K_d} \right)$$ where v is the reaction rate, $k_{cat}$ is catalytic rate constant, L is the concentration of LPL.

Figure 5c is the concentration of apoC-II, β is the activation factor which indicates how much more active the LPL/apoC-II complex is compared to LPL alone, Kd is the equilibrium dissociation constant.

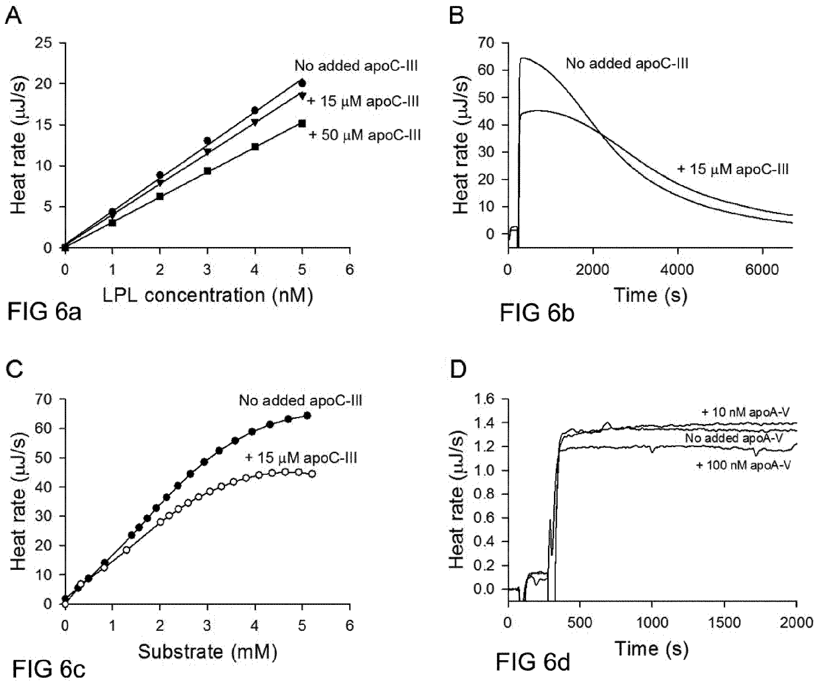

Figure 6a and figure 6b illustrate the effect of apoC-III and apoA-V on LPL activity in plasma.

Figure 6a *illustrates* effect of apoC-III on LPL activity under zero-order reaction conditions. Sequential injections of LPL were made into plasma samples in which apoC-III concentration was increased by 15 $\mu$M, 50 $\mu$M or in alternative embodiment of present invention not increased. Each injection increased LPL concentration by 1 nM. Concentration of triglycerides in plasma was 2 mM.

Figure *6b illustrates* total hydrolysis thermograms of plasma triglycerides by LPL in the case when plasma concentration of apoC-III was not increased or in alternative embodiment of present invention was increased by 15 $\mu$M. Concentration of LPL was 22 nM and concentration of triglycerides was 2.7 mM. An example of one measurement from three different measurements is presented for both cases.

Figure 6c illustrates dependence of heat rate on triglyceride concentration as calculated from the total hydrolysis curves presented in figure 6b.

Figure 6d illustrates LPL activity, which was measured in the absence or in alternative embodiment of present invention in the presesnce of 10 nM or 100 nM apoA-V. Single injection of LPL into the plasma was made. TG and LPL concentration in the ITC cell were 1 mM and 0.6 nM, respectively.

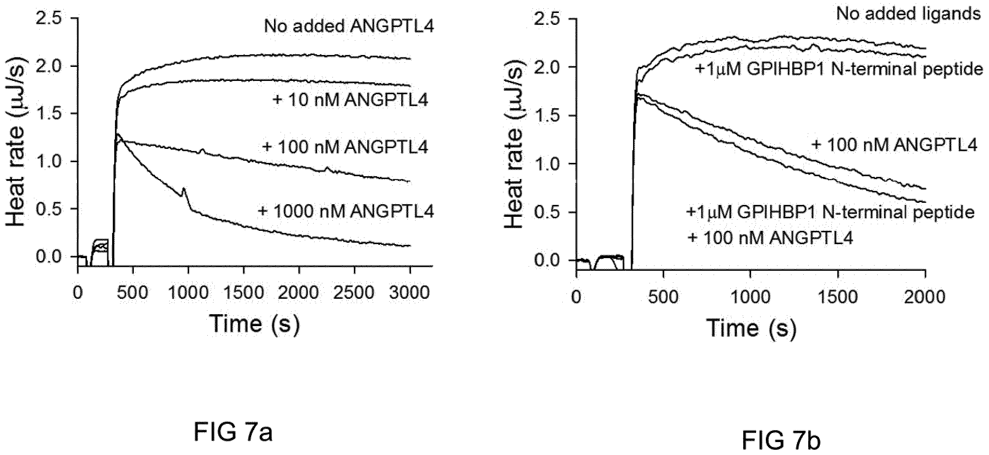

Figure 7a to figure 7c illustrate effect of ANGPTL4 or ANGPTL3 on LPL activity.

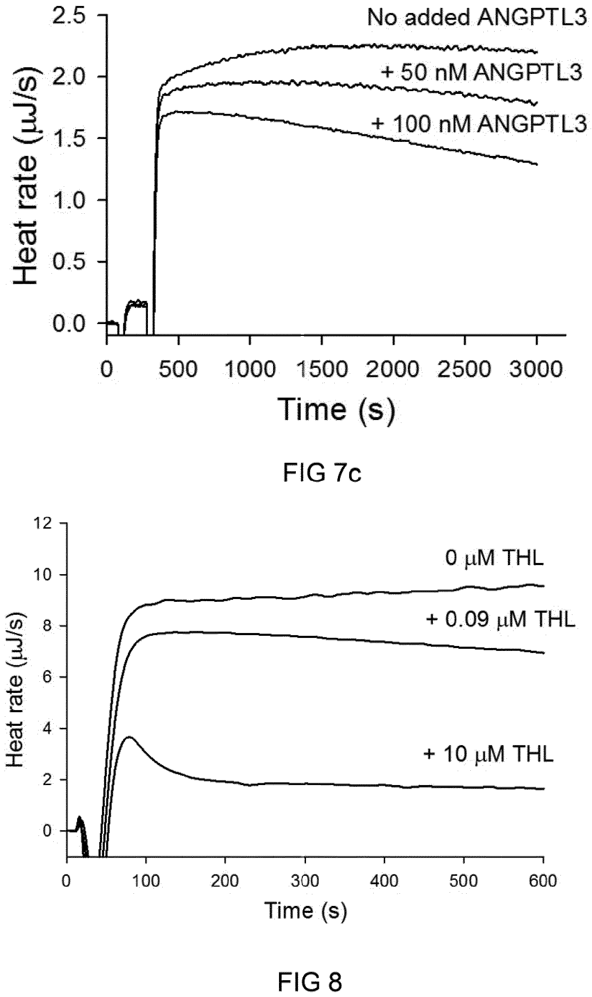

*Figure 7a* and *figure 7c* illustrates LPL activity, which was measured in the absence or in alternative embodiment of present invention presence of different concentrations of ANGPTL4 (A) or ANGPTL3 (C).

Figure 7b illustrates ANGPTL4 effect on LPL, which was tested in the presence of the N-terminal peptide of GPIHBP1. In all experiments single injection of LPL into plasma sample was made; resulting concentration of LPL in the ITC cell was 0.6 nM. Triglyceride concentration was 1 mM.

Figure 8 illustrates effect of tetrahydrolipstatin (THL) on LPL activity, wherein single injection of 10 $\mu$l of 100 nM LPL into 1 ml of human plasma that contained or in alternative embodiment of present invention did not contain THL. Final concentration of LPL was 1 nM.

## Detailed description of the invention

The method for calorimetric determination of the lipoprotein lipase activity in human plasma according to present invention comprises steps of preparing at least one reagent, obtaining at least one decoded human plasma sample, preparing the sample, performing isothermal titration calorimetry (ITC) measurements, wherein ITC measurements are used for measuring LPL activity in plasma, recording total hydrolysis of plasma lipids by LPL and to recording differences in how well LPL can get access to the lipids, determing the influence of added regulators on the LPL reaction in plasma.

More specifically, the method according to present invention for calorimetric determination of the lipoprotein lipase activity in human plasma comprises steps of:

- preparing at least one reagent,
- obtaining at least one human plasma sample,
- preparing the human plasma sample,
- filling the isothermal titration calorimetry cell withe prepared human plasma sample,
- injecting LPL to the cell from the syringe-stirrer,
- changing the heat rate as a result of injection of LPL into ITC cell with human plasma;
- increasing LPL concentration in the ITC cell by at least one injection;
- performing isothermal titration calorimetry measurements, and said ITC measurements are used for measuring LPL activity in human plasma,
- recording the results.

The heat rate is changed as a result of single injection or in an alternative embodiment as a result of sequential injection of LPL into ITC cell with human plasma. The heat rate is changed proportionally to the reaction rate, as a result of injection of LPL into ITC cell with 1ml human plasma, as a result of injection of LPL into ITC cell with human plasma substrate mixture containing human VLDL, as a result of injection of LPL into ITC cell with human plasma substrate mixture containing Intralipid, as a result of injection of LPL into ITC cell with human postheparin plasma mixture containing Anti-HL IgG in 1 ml of human pre-heparin plasma, as a result of injection of LPL into ITC cell with human post-heparin plasma mixture in 1 ml of human pre-heparin plasma.

By recording the results, total hydrolysis of plasma lipids by LPL, differences in how well LPL can get access to the lipids, how LPL can catalyse hydrolysis of lipids in plasma samples is recorded as the results or the influence of added regulators on the LPL reaction in plasma is recorded as the results.

*Preparing the reagents* - Bovine LPL was purified from milk (Bengtsson-Olivecrona & Olivecrona 1991) and dialysed to buffer containing 10 mM TRIS, pH 8.5 (4°C), 4 mM sodium deoxycholate. Stock solutions of 0.5 mg/ml LPL were stored at -80°C. The N-terminal coiled-coil domain of human angiopoietin-like protein 4 (ANGPTL4), residues 26-184, was expressed in *E. coli* and purified as described (Robal et al. 2012). Full-length human angiopoietin-like protein 3 (ANGPTL3) expressed in Sf 21 cells was obtained from R&D Systems (USA). Apolipoprotein C-III$_0$ was from human blood plasma (Bengtsson-Olivecrona & Sletten 1990). Apolipoprotein C-II and apolipoprotein A-V were expressed in *E. coli* and purified as described (Shen et al. 2010; Beckstead et al. 2003). A synthetic peptide corresponding to the N-terminal domain of human GPIHBP1, residues 23-51, was bought from Caslo (Denmark). The sequence of the peptide was as follows: QQEEEEEDEDHGPDDYDEEDEDEVEEEET. Antibodies (goat IgG) to human hepatic lipase were raised in a goat against hepatic lipase isolated from human postheparin plasma (Olivecrona & Olivecrona 2000). The IgG fraction was isolated using Protein-A columns and the final preparation contained 5 mg protein per milliliter in 20 mM Na-phosphate buffer and 0.15 M NaCl (pH 7.4).

Decoded human plasma samples (treated by EDTA) were obtained from the Blood Centrum or were obtained from blood taken by forearm vein puncture from healty 20-30 years old volunteers 2 hours after they had eaten a normal meal. Cells were removed from plasma by centrifugation for 30 minutes at 2000 x g at 4°C. The plasma samples were aliquoted and stored at -80°C and were generally only used once. EnzyChrom Triglyceride Assay Kit (BioAssay Systems, USA) or Triglyceride Colorimetric Assay Kit (Cayman, USA) were used for determination of triglyceride concentrations. Free fatty acids were quantified by the NEFA-HR(2) kit (Wako Chemicals). A standard sample of human post-heparin plasma (used for many years in the gorup at Umeå University) was from a male volunteer that had recieved 100 IU heparin/kg body weight by i.v. injection in one forearm. After 15 min blood was collected into heparinized tubes from the other arm and plasma was collected by centrifugation (Human PHPL st 23) (Karpe et al. 1992; Tornvall et al. 1995). Human very low density lipoproteins (VLDL) were purified from normal human plasma by ultracentrifugation (Havel et al. 1955). The final preparation contained 1.3 mg protein/ml and 2.5 mM triglycerides. Some human VLDL were purchased from Kalen Biomedical (USA). This preparation contained 1 mg protein/ml and 4.2 mM triglycerides. Goat serum was from Invitrogen (product code 10000C). Intralipid (a 20% phospholipid-stabilized emulsion of soy bean triglycerides used for parenteral nutrition of patients) was obtained from Sigma. Heparin was purchased from LEO Pharma (Denmark)

*Sample preparation* - Before experiments the human plasma samples were diluted 1.2 times with TRIS buffer pH 7.4 or with the additions specified for each experiment. The final concentration of TRIS was 20 mM in all cases. Stock solutions of LPL were diluted in cold 10 mM TRIS, pH 8.5, containing 4 mM sodium deoxycholate. In this buffer, LPL is stable for a long period of time, even at low protein concentrations The final concentration of deoxycholate during incubations with plasma or lipoproteins was 10 to 100 times lower than the initial. Control experiments showed that these levels had no influence on the enzymatic reaction. In experiments with post-heparin plasma, antibodies to hepatic lipase (in PBS) or the same volume of PBS only were added (180 $\mu$l of IgG or PBS was mixed with 360 $\mu$l of post-heparin plasma) and the mixture was then incubated for 2 hours on ice prior to experiments (Olivecrona & Olivecrona 2000). All samples were degassed under vacuum for 15 minutes before the ITC experiments.

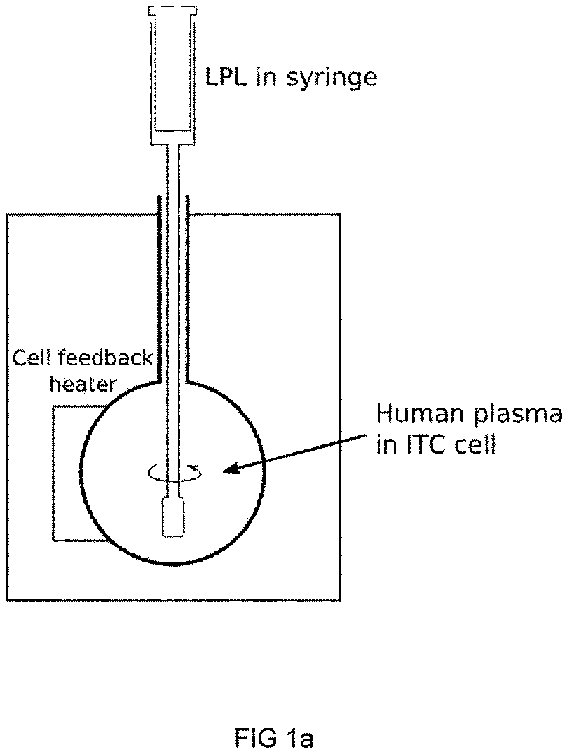

*ITC measurements* - Most of the experiments were performed on an Nano ITC model 5300 (TA Instruments, USA) at 25C. A MicroCal Auto-iTC200 (GE Healthcare) instrument was used for experiments regarding relationship between total heat production and released fatty acids. In a typical experiment performed by Nano ITC, the lipase substrate (plasma, Intralipid 20% or VLDL) with or without added ligands was placed into the calorimetric cell (1035 $\mu$l) and the syringe (250 $\mu$l) was filled with LPL-containing solution (bovine LPL or post-heparin plasma) (see Figure 1A). MilliQ water was loaded into the reference cell (1032 $\mu$l). The stirring speed in the sample cell was 400 rpm. The baseline was left to stabilize for at least 1 hour before injections of LPL or post-heparin plasma. In all experiments, the first injection was 2 $\mu$l and after that single or sequential injections of 10 to 25 $\mu$l were made. The interval between the injections was from 200 s to 500 s. In experiments performed by Micro-Cal Auto-iTC200, 400 $\mu$l of lipase substrate (plasma), 400 $\mu$l of equilibration buffer (20 mM TRIS, pH 7,4) and a sufficient volume of LPL (0.8 $\mu$M) were placed on the loading plate. The calorimetric cell (200 $\mu$l) was rinsed with equilibration buffer and loaded with 200 $\mu$l of substrate in an automated fashion. The syringe was loaded with 40 $\mu$l of LPL-containing solution. The stirring speed in the sample cell was 600 rpm and the baseline was stabilized automatically. The first injection of LPL was 0.2 $\mu$l and the second injection was 2 $\mu$l. The experiment time was 1 hour to allow full hydrolysis of plasma triglycerides.After each experiment the Nano ITC sample cell was washed with the following solutions, one after the other: MilliQ water, 2% SDS, 40 mM NaOH and finally 95% ethanol. The MicroCal Auto-iTC200 was washed with MilliQ water, 10% Decon90 (Decon Laboratories Ltd.), and 100% methanol. Raw ITC data were analysed using the NanoAnalyze (TA Instruments) Or MicroCal Origin (GE Healthcare).

The following examples and experiments are useful for understanding and highlighting specific aspects of the present invention and therefore they are present for illustrating purposes only.

## ITC can be used for measurements of LPL activity in plasma

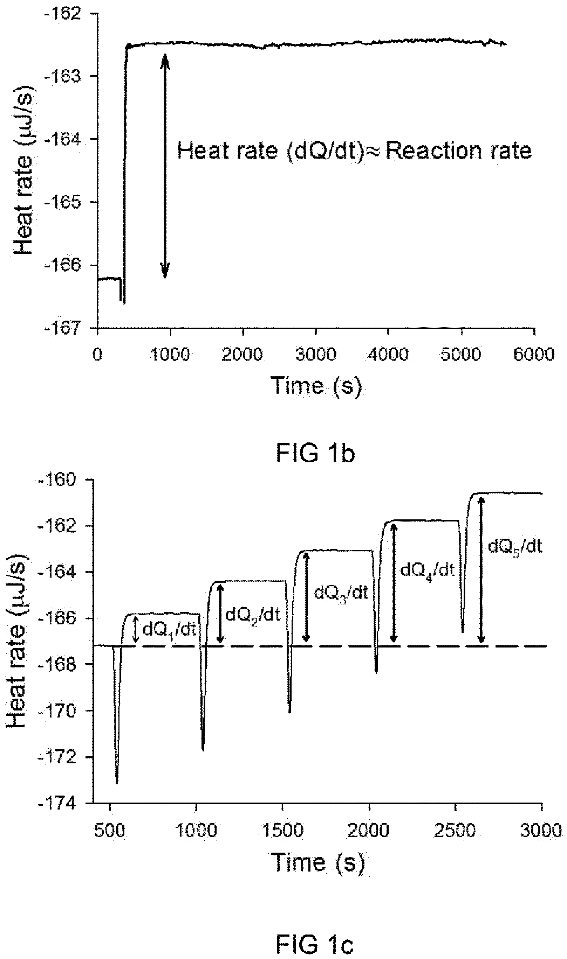

To evaluate whether ITC can be used for studies of LPL, we first tested the stability of LPL during the ITC experiments. The ITC cell was filled with normal human plasma and a diluted sample of LPL was injected from the syringe (Figure 1A). As can be seen in Figure 1B, this resulted in an increase of the heat rate that remained constant for the duration of the experiment (5500 seconds). This demonstrated, as expected, that the reaction was exothermic, but also, and less expected, that the catalytic activity of LPL was unchanged during the whole experiment. The reaction followed zero-order kinetics, meaning that consumption of the substrate (presumably triglycerides and phospholipids in plasma lipoproteins), or changes of the physical properties of the substrate, did not influence on the reaction rate. Sequential injections of LPL into the ITC cell with plasma led to a step-wise increase of the heat rate (Figure 1C). The heat rate level increased almost equally by each injection of LPL, indicating a proportional relationship between the concentration of LPL and the reaction rate (Figure 1D). As expected, the standard deviations of the slopes calculated from sequential injections were smaller than that of increments of heat rate level after single injections. The difference of the two standard deviations was about two times. Thus, it was preferable to measure LPL activity using the sequential injection mode.

Next the activity of LPL in plasma is compared to recorded with isolated VLDL or with the synthetic lipid emulsion Intralipid (Figure 2). Using the same initial triglyceride concentration (1 mM), the heat rate level was proportional to the LPL concentration at least up to 400 pM in all tested systems (Figure 2). The observed heat production rate was almost the same in all three systems, suggesting that the total heat production is mainly due to hydrolysis of triglycerides. The lowest concentration of LPL detectable by ITC varied only slightly between the substrate systems. It is generally accepted that a measurement is reliable when determined parameter is at least ten times over the noise level. The noise level calculated as standard deviation of the heat rate level was found to be $24.6 \pm 2.1$ nJ/s. Hence, for reliable determination of LPL activity the change of heat rate must be over 250 nJ/s. Based on the slope of the relationship between heat rate and LPL concentration (Figure 2, human plasma), it was possible to determine that 50 pM LPL is the lowest concentration that can be reliably measured by the ITC instrument when the triglyceride concentration is 1 mM. The broad linearity range demonstrates that the ITC technique is suitable for LPL activity measurements.

LPL in its active form is normally not present in blood, but is attached to capillary walls in a heparin-releasable manner. Therefore i.v. injection of heparin is often used to release LPL from its binding sites into the circulating blood. The amount of LPL in post-heparin plasma gives an estimate of the total pool of LPL that have access to lipoprotein in plasma and is often designated the functional pool of LPL in contrast to LPL contained within cells and the extracellular matrix (Tornvall et al. 1995). To investigate whether ITC could be used for measurement of LPL activity in postheparin plasma, we injected small amounts of human post-heparin plasma from the syringe into the ITC cell containing triglyceride-rich, normal human plasma as substrate. Like in the case with purified bovine LPL, a linear relationship between the amount of post-heparin plasma and the heat rate level was observed (Figure 3A). Said experiment involving injection of post-heparin plasma is not part of the invention. Post-heparin plasma is known to contain another lipase in addition to LPL, named hepatic lipase (HL) based on its tissue origin (Perret et al. 2002). Therefore, a sample of post-heparin plasma is treated with antibodies known to specifically inactivate hepatic lipase (Anti-HL IgG) (Tornvall et al. 1995; Olivecrona & Olivecrona 2000). This only slightly affected the slope of the heat rate versus plasma volume. The t-test analysis revealed that the differences between the slopes of the two lines (with and without the inhibitory antibody) were not significantly different (P=0.13; paired, two-tailed distribution). Thus, the detected activity in post-heparin plasma can be almost completely attributed to LPL. Based on comparison of the activity of purified bovine LPL with that of post-heparin plasma LPL (Figures 2, human plasma and 3A), it is estimated that the sample of post-heparin plasma should contain 7.3 pmol LPL/ml. To devise an ITC assay for LPL for general use, measurements were performed with a commercial preparation of VLDL (Figure 3B). Due to the low triglyceride concentration in this preparation, the activity of LPL was lower than in Figure 3A (about one forth), but still sufficiently high for a linear determination of LPL activity based on increased additions of post-heparin plasma to the ITC cell.

**ITC can be used to record total hydrolysis of plasma lipids by LPL and to record differences in how well LPL can get access to the lipids.** In the next experiments, ITC was used for monitoring of the total hydrolysis of lipids in plasma by LPL. For practical registration of total hydrolysis in real time, the concentration of LPL used was 40-100 times higher than that used for determination of initial rates (Figure 1B and 1C). Examples of total hydrolysis curves (run in triplicates) for two plasma samples that differed in their initial triglyceride concentrations (1 mM and 2.7 mM, respectively) are shown in Figure 4A. The areas under these curves correspond to the total heat production. They differed 2.6 times, indicating a good correlation between total heat production and the initial triglyceride concentration of the plasma samples. The concentration of released fatty acids determined by the NEFA kit at the end of each reaction was approximately two times higher than the initial triglyceride concentration. This is in agreement with that LPL is known to catalyze hydrolysis of the ester bonds only at positions sn1 and sn3 of triglycerides (Olivecrona & Olivecrona 2009), and with that isomerization of acyl groups from the sn 2 position to the sn 1(3) positions is slow at pH 7.4. Thus, it is reasonable to assume that the real substrate concentration is equal to the concentration of hydrolysable ester bonds which is two times higher than the triglyceride concentration. Using results from several experiments with different plasma samples, a linear correlation was found between the total heat production and the amount of formed fatty acids (Figure 4B). Since this relationship included data from lipolysis of various depth, it was reasonable to consider that the total heat effect is equally determined by hydrolysis of ester bonds of triglycerides and diglycerides. The relationship also suggests that the heat effect of structural changes and reorganization of lipoproteins during the lipolysis is negligible in comparison of that of the hydrolysis of ester bonds. To further analyze the total hydrolysis curves in Figure 4A, the raw data were transformed into a plot of reaction rate versus remaining substrate concentration (Figure 4C). This was obtained by subtracting the hydrolyzed substrate concentration at a chosen time point from the total substrate concentration (the total area). The calculations were based on the assumption that the substrate concentration was equal to concentration of hydrolysable ester bonds that was in turn two times higher than the triglyceride concentration. This transformation enabled us to examine how the reaction rate depended on the substrate concentration using a single hydrolysis curve. As can be seen, the reaction rate for these samples differed when the substrate concentration in plasma was high, but the rates were overlapping in the lower substrate range. This result indicate that there might be detectable differences in how good access LPL has to lipid substrates in different plasma samples during total hydrolysis.

**ITC can be used to study the influence of added regulators on the LPL reaction in plasma**

In the next set of experiments, it is examined how the LPL activity on plasma lipoproteins was influenced by the regulator proteins apoC-II, apoC-III, apo A-V, ANGPTL3 and ANGPTL4. Experiments with the activator apoC-II were carried out using Intralipid as substrate, because plasma normally contains sufficient apoC-II for full activation of LPL (Kei et al. 2012). In these measurements, the LPL concentration was held constant while the apoC-II concentration varied from 0 to 1000 nM. Raw ITC thermograms are presented in Figure 5A. As expected, increased concentrations of apoC-II caused increased activity of LPL. To estimate the activation factor and the affinity of apoC-II for LPL, the heat rate values at 200 s were plotted against the apoC-II concentration (Figure 5B). As can be seen, apoC-II activated LPL in a saturable fashion. For the analysis of the apoC-II activation data, we used a model proposed by Quinn, Shirai, and Jackson (Quinn et al. 1983). The estimated maximal activation factor was $4.2 \pm 1.2$ and the $K_d$ value was $22 \pm 8$ nM. The real time recordings demonstrated that LPL was not stable in the absence of apoC-II, or when its concentration was lower than 100 nM. Thus, in addition to activation apoC-II also stabilized LPL. The stabilization effect of apoC-II is usually not detectable in other substrate systems. The continuous monitoring of the reaction rate by ITC provides more detailed information about the first minutes of the reaction.

The effect of the inhibiting protein apoC-III on LPL activity was studied after addition to plasma under two experimental circumstances: 1) under zero order conditions when the LPL concentration was so low that the reaction rate was constant during the reaction time (zero-order conditions - depletion of substrates did not influence the reaction rate) 2) in the case of total hydrolysis of the available substrates in plasma with about 20-fold more LPL to allow monitoring in real time. Under the zero-order conditions the reduction of the reaction rate was observed at the starting of the reaction and remained unchanged for more than one hour (data not shown). This suggested that apoC-III affected the LPL activity, but not the stability of LPL in plasma. Figure 6A presents results of sequential injection of LPL into plasma samples under zero-order conditions without additional apoC-III and after increasing the apoC-III concentration by 15 or 50 $\mu$M. From these data it is evident that apoC-III somehow restricts the activity of LPL. In Figure 6B the effect of addition of apoC-III to an additional concentration of 15 $\mu$M on the time course for total lipolysis in plasma is shown. This means about a doubling of the concentration of apoC-III in normal human plasma (Fredenrich et al. 1997). The initial inhibiting effect on heat rate production is clearly seen, while with time, when most of the available substrate had been degraded, the rates became similar. After transformation of the raw ITC data into a plot of reaction rate versus remaining triglyceride concentration, it became evident that apoC-III inhibited LPL only when lipoproteins with high triglyceride concentrations were available (Figure 6C).

Animal experiments (Pennacchio et al. 2001) and populations studies in humans (Do et al. 2015; Anon 2010) have demonstrated that ApoA-V is an important regulator of triglyceride levels in plasma. ApoAV lowers triglyceride concentrations *in vivo,* but its effect has been difficult to reproduce *in vitro* (Nilsson et al. 2011). Although many evidence point to a stimulation of the activity of LPL by apoA-V, the detailed mechanism is still unknown. In ITC experiments low concentrations of apoAV (10 nM) did not change the activity of LPL (Figure 6D). On the contrary, at higher concentrations of apoA-V (100 nM), some inhibition was observed. It should be noted that the normal concentration of apoA-V in plasma is much lower than for apoC-III, about 4 nM (O'Brien et al. 2005).

Studies in recent years have identified some of the angiopoietin-like proteins (ANGPTLs) as important regulators of lipid metabolism and in particular as inhibitors/inactivators of LPL (Dijk & Kersten 2016). The normal range for ANGPTL4 in plasma is between 0.04 and 3 nM (Robciuc et al. 2010). In experiments with ITC some suppression of the LPL activity in plasma by ANGPTL4 (the N-terminal fragment) was observed from the start of the lipolysis, immediately after injection of LPL. A 10% drop of LPL activity was detected when the ANGPTL4 concentration was increased in plasma by only 10 nM (Figure 7A). With much higher concentrations of added ANGPTL4 fragment (100 nM or 1000 nM), the activity of LPL initially dropped more and continued to decrease after the first drop (Figure 7A). These observations suggest that ANGPTL4 may affect LPL activity in plasma through two different mechanisms, one immediate that may involve inhibition of activity, but likely not to irreversible inactivation. The other, seen at higher concentrations of ANGPTL4, is likely reflecting continuous, irreversible inactivation of LPL. This occurs even though the enzyme is very stable in plasma under the conditions of ITC and the measurements performed under the zero order conditions, when decreased availability of substrates should not influence the activity of LPL. Others have demonstrated that the endothelial protein GPIHBP1, a transporter protein for LPL, can stabilize LPL against inactivation by ANGPTL4 (Sonnenburg et al. 2009). The exceptionally acidic N-terminal peptide of GPIHBP1, known to bind and stabilize LPL under other conditions (Reimund et al. 2015), could not protect LPL from inactivation by 100 nM ANGPTL4 under the conditions for ITC (Figure 7B). The full-length ANGPTL3 had similar effects as the N-terminal fragment of ANGPTL4 (Figure 7C).

To test whether ITC is a suitable technique for investigations of LPL inhibitors, experiments with THL that is an active site inhibitor of LPL and other lipases are conducted (Lookene et al. 1994). THL reduced the activity of LPL in plasma in a concentration dependent manner, while the inhibition was detectable already at 90 nM concentration of THL (Figure 8). This indicates high specificity of the LPL/THL interaction, since the LPL concentration in this plasma sample was very low in comparison to abundant plasma proteins. In the presence of 10 $\mu$M THL, the activity of LPL dropped immediately 80% but remained after that unchanged. The absence of inhibition kinetics in this case suggests that not all of LPL in plasma is accessible for THL. Surprisingly, poloxamer 407, an inhibitor of LPL in mice (Johnston 2010), did not affect the LPL activity in human plasma even at concentration 50 $\mu$M.

## REFERENCES

Anon, 2010. Triglyceride-mediated pathways and coronary disease: collaborative analysis of 101 studies. The Lancet, 375(9726), pp.1634-1639.

Basu, D., Manjur, J. & Jin, W., 2011. Determination of lipoprotein lipase activity using a novel fluorescent lipase assay. Journal of lipid research, 52(4), pp.826-32.

Beckstead, J.A. et al., 2003. Structure-function studies of human apolipoprotein A-V: a regulator of plasma lipid homeostasis. Biochemistry, 42(31), pp.9416-23.

Bengtsson-Olivecrona, G. & Olivecrona, T., 1991. Phospholipase activity of milk lipoprotein lipase. Methods in enzymology, 197, pp.345-56.

Bengtsson-Olivecrona, G. & Sletten, K., 1990. Primary structure of the bovine analogues to human apolipoproteins CII and CIII. Studies on isoforms and evidence for proteolytic processing. European journal of biochemistry / FEBS, 192(2), pp.515-21.

Bianconi, M.L., 2005. Titration Calorimetry as a Tool to Determine Thermodynamic and Kinetic Parameters of Enzymes. In J. E. Ladbury & M. L. Doyle, eds. Biocalorimetry 2. Chichester, UK: John Wiley & Sons, Ltd, pp. 175-185.

Briquet-Laugier, V., Ben-Zeev, O. & Doolittle, M.H., 1999. Determining lipoprotein lipase and hepatic lipase activity using radiolabeled substrates. Methods in molecular biology (Clifton, N.J.), 109, pp.81-94.

Dallinga-Thie, G.M. et al., 2016. Triglyceride-Rich Lipoproteins and Remnants: Targets for Therapy? Current cardiology reports, 18(7), p.67.

Dijk, W. & Kersten, S., 2016. Regulation of lipid metabolism by angiopoietin-like proteins. Current opinion in lipidology, 27(3), pp.249-56.

Do, R. et al., 2015. Exome sequencing identifies rare LDLR and APOA5 alleles conferring risk for myocardial infarction. Nature, 518(7537), pp.102-6.

Dole, V., 1956. A relation between non-esterified fatty acids in plasma and the metabolism of glucose. The Journal of clinical investigation, 35(2), pp.150-4.

Eisenberg, S. & Olivecrona, T., 1979. Very low density lipoprotein. Fate of phospholipids, cholesterol, and apolipoprotein C during lipolysis in vitro. Journal of lipid research, 20(5), pp.614-23.

Ellis, R.J., 2001. Macromolecular crowding: obvious but underappreciated. Trends in Biochemical Sciences, 26(10), pp.597-604.

Fredenrich, A. et al., 1997. Plasma lipoprotein distribution of apoC-III in normolipidemic and hypertriglyceridemic subjects: comparison of the apoC-III to apoE ratio in different lipoprotein fractions. Journal of lipid research, 38(7), pp.1421-32.

Havel, R.J., Eder, H.A. & Bragdon, J.H., 1955. The distribution and chemical composition of ultracentrifugally separated lipoproteins in human serum. The Journal of clinical investigation, 34(9), pp.1345-53.

Johnston, T.P., 2010. Poloxamer 407 as a general lipase inhibitor: its implications in lipid metabolism and atheroma formation in C57BL/6 mice. The Journal of pharmacy and pharmacology, 62(12), pp.1807-12.

Karpe, F. et al., 1992. Lipoprotein lipase in plasma after an oral fat load: relation to free fatty acids. Journal of lipid research, 33(7), pp.975-84.

Kei, A.A. et al., 2012. A review of the role of apolipoprotein C-II in lipoprotein metabolism and cardiovascular disease. Metabolism: clinical and experimental, 61(7), pp.906-21.

Khetarpal, S.A. & Rader, D.J., 2015. Triglyceride-rich lipoproteins and coronary artery disease risk: new insights from human genetics. Arteriosclerosis, thrombosis, and vascular biology, 35(2), pp. e3-9.

Lafferty, M.J. et al., 2013. Angiopoietin-like protein 4 inhibition of lipoprotein lipase: evidence for reversible complex formation. The Journal of biological chemistry, 288(40), pp.28524-34.

Larsson, M. et al., 2013. Apolipoproteins C-I and C-III inhibit lipoprotein lipase activity by displacement of the enzyme from lipid droplets. The Journal of biological chemistry, 288(47), pp.33997-4008.

Lookene, A. et al., 2004. Rapid subunit exchange in dimeric lipoprotein lipase and properties of the inactive monomer. The Journal of biological chemistry, 279(48), pp.49964-72.

Lookene, A. & Bengtsson-Olivecrona, G., 1993. Chymotryptic cleavage of lipoprotein lipase. Identification of cleavage sites and functional studies of the truncated molecule. European journal of biochemistry / FEBS, 213(1), pp.185-94.

Lookene, A., Savonen, R. & Olivecrona, G., 1997. Interaction of lipoproteins with heparan sulfate proteoglycans and with lipoprotein lipase. Studies by surface plasmon resonance technique. Biochemistry, 36(17), pp.5267-75.

Lookene, A., Skottova, N. & Olivecrona, G., 1994. Interactions of lipoprotein lipase with the active-site inhibitor tetrahydrolipstatin (Orlistat) R. , 403, pp.395-403.

Musunuru, K. & Kathiresan, S., 2016. Surprises From Genetic Analyses of Lipid Risk Factors for Atherosclerosis. Circulation research, 118(4), pp.579-85.

Nilsson, S.K. et al., 2011. Apolipoprotein A-V; a potent triglyceride reducer. Atherosclerosis, 219(1), pp.15-21.

Nordestgaard, B.G., 2016. Triglyceride-Rich Lipoproteins and Atherosclerotic Cardiovascular Disease: New Insights From Epidemiology, Genetics, and Biology. Circulation research, 118(4), pp.547-63.

O'Brien, P.J. et al., 2005. The novel apolipoprotein A5 is present in human serum, is associated with VLDL, HDL, and chylomicrons, and circulates at very low concentrations compared with other apolipoproteins. Clinical chemistry, 51(2), pp.351-9.

Olivecrona, G., 2016. Role of lipoprotein lipase in lipid metabolism. Current opinion in lipidology, 27(3), pp.233-41.

Olivecrona, G. & Beisiegel, U., 1997. Lipid binding of apolipoprotein CII is required for stimulation of lipoprotein lipase activity against apolipoprotein CII-deficient chylomicrons. Arteriosclerosis, thrombosis, and vascular biology, 17(8), pp.1545-9.

Olivecrona, T. & Olivecrona, G., 2000. Determination and Clinical Significance of Lipoprotein Lipase and Hepatic Lipase. In N. Rifai, G. R. Warnick, & M. H. Dominiczak, eds. Handbook of Lipoprotein Testing. Washington DC: AACC Press, pp. 479-498.

Olivecrona, T. & Olivecrona, G., 2009. The Ins and Outs of Adipose Tissue. In C. Ehnholm, ed. Cellular Lipid Metabolism. Springer Berlin Heidelberg, pp. 315-369.

Panteghini, M., Bonora, R. & Pagani, F., 2001. Measurement of pancreatic lipase activity in serum by a kinetic colorimetric assay using a new chromogenic substrate. Annals of clinical biochemistry, 38(Pt 4), pp.365-70.

Pennacchio, L.A. et al., 2001. An apolipoprotein influencing triglycerides in humans and mice revealed by comparative sequencing. Science (New York, N.Y.), 294(5540), pp.169-73.

Perret, B. et al., 2002. Hepatic lipase: structure/function relationship, synthesis, and regulation. Journal of lipid research, 43(8), pp.1163-9.

Peterson, J. et al., 1990. Fatty acid control of lipoprotein lipase: a link between energy metabolism and lipid transport. Proceedings of the National Academy of Sciences of the United States of America, 87(3), pp.909-13.

Quinn, D., Shirai, K. & Jackson, R.L., 1983. Lipoprotein lipase: mechanism of action and role in lipoprotein metabolism. Progress in lipid research, 22(1), pp.35-78.

Rader, D.J., 2016. New Therapeutic Approaches to the Treatment of Dyslipidemia. Cell Metabolism, 23(3), pp.405-412.

Reimund, M. et al., 2015. Evidence for Two Distinct Binding Sites for Lipoprotein Lipase on Glycosylphosphatidylinositol-anchored High Density Lipoprotein-binding Protein 1 (GPIHBP1). The Journal of biological chemistry, 290(22), pp.13919-34.

Robal, T. et al., 2012. Fatty acids bind tightly to the N-terminal domain of angiopoietin-like protein 4 and modulate its interaction with lipoprotein lipase. Journal of Biological Chemistry, 287(35), pp.29739-29752.

Robciuc, M.R. et al., 2010. Quantitation of serum angiopoietin-like proteins 3 and 4 in a Finnish population sample. Journal of lipid research, 51(4), pp.824-31.

Shan, L. et al., 2009. The angiopoietin-like proteins ANGPTL3 and ANGPTL4 inhibit lipoprotein lipase activity through distinct mechanisms. The Journal of biological chemistry, 284(3), pp.1419-24.

Shen, Y. et al., 2010. Site-directed Mutagenesis of Apolipoprotein CII to Probe the Role of Its Secondary Structure for Activation of. , 285(10), pp.7484-7492.

Sonnenburg, W.K. et al., 2009. GPIHBP1 stabilizes lipoprotein lipase and prevents its inhibition by angiopoietin-like 3 and angiopoietin-like 4. Journal of lipid research, 50(12), pp.2421-2429.

Sukonina, V. et al., 2006. Angiopoietin-like protein 4 converts lipoprotein lipase to inactive monomers and modulates lipase activity in adipose tissue. Proceedings of the National Academy of Sciences of the United States of America, 103(46), pp.17450-5.

Tornvall, P. et al., 1995. Lipoprotein lipase mass and activity in plasma and their increase after heparin are separate parameters with different relations to plasma lipoproteins. Arteriosclerosis, thrombosis, and vascular biology, 15(8), pp.1086-93.

## Claims

1. A method for calorimetric determination of the lipoprotein lipase (LPL) activity in human plasma comprising steps of:

   - preparing at least one reagent containing LPL by purifying bovine LPL from milk and dialyzing it to TRIS buffer solution containing 4mM sodium deoxycholate and having pH 8.5 at 4°C, - introducing a plasma sample obtained from a human,
   - preparing the human plasma sample from blood samples by removing cells by centrifugation for 30 min at 2000 x g at 4 °C,
   - filling the isothermal titration calorimetry cell with the prepared human plasma sample,
   - injecting reagent containing LPL to the ITC cell from the syringe-stirrer,
   - changing the heat rate as a result of injection of LPL into Isothermal Titration Calorimeter (ITC) cell with human plasma;
   - increasing LPL concentration in the ITC cell by at least one injection;
   - performing isothermal titration calorimetry measurements, wherein ITC measurements are used for measuring LPL activity in human plasma,
   - recording the results.

2. The method according to claim 1, wherein the heat rate is changed as a result of single injection of LPL into ITC cell with human plasma.

3. The method according to claim 1, wherein the heat rate is changed as a result of sequential injections of LPL into ITC cell with human plasma.

4. The method according to claim 1, wherein the heat rate is changed proportionally to the reaction rate.

5. The method according to claim 1, wherein the heat rate is changed as a result of injection of LPL into ITC cell with 1ml human plasma.

6. The method according to claim 1, wherein the heat rate is changed as a result of injection of LPL into ITC cell with human plasma substrate mixture containing human VLDL.

7. The method according to claim 1, wherein the heat rate is changed as a result of injection of LPL into ITC cell with human plasma substrate mixture containing a 20% phospholipid stabilized emulsion of soy bean triglycerides

8. The method according to claim 1, wherein the concentration of LPL in plasma is 22 nM.

9. The method according to claim 1, wherein the concentration of triglycerides in plasma is 2.7 mM.

10. The method according to claim 1, wherein LPL concentration is increased in the ITC cell by each injection by 50 pM.

11. The method according to claim 1, wherein total hydrolysis of plasma lipids by LPL is recorded as the results.

12. The method according to claim 1, wherein differences in how well LPL can get access to the lipids is recorded as the results.

13. The method according to claim 1, wherein how LPL can catalyse hydrolysis of lipids in plasma samples is recorded as the results.

14. The method according to claim 1, wherein the influence of added regulators on the LPL reaction in plasma is recorded as the results.

**Patentansprüche**

1. Verfahren zur kalorimetrischen Bestimmung der Lipoproteinlipase (LPL)-Aktivität in menschlichem Plasma, umfassend die folgenden Schritte:

   - herstellen mindestens eines LPL-haltigen Reagenzes durch Reinigen von Rinder-LPL aus Milch und Dialysieren desselben in eine TRIS-Pufferlösung, die 4 mM Natriumdesoxycholat enthält und bei 4 °C einen pH-Wert von 8,5 aufweist,
   - einbringen einer von einem Menschen gewonnenen Plasmaprobe,
   - herstellen der menschlichen Plasmaprobe aus Blutproben durch Entfernen von Zellen durch Zentrifugieren für 30 Minuten bei 2000 x g bei 4 °C,
   - befüllen der isothermen Titrationskalorimetriezelle mit der hergestellten menschlichen Plasmaprobe,
   - injizieren von LPL enthaltendem Reagenz aus dem Spritzenrührer in die ITC-Zelle,
   - ändern der Wärmerate infolge der Injektion von LPL in die isotherme Titrationskalorimeterzelle (ITC-Zelle) mit menschlichem Plasma;
   - erhöhen der LPL-Konzentration in der ITC-Zelle durch mindestens eine Injektion;
   - durchführen von isothermen Titrationskalorimetriemessungen, wobei ITC-Messungen zur Messung der LPL-Aktivität in menschlichem Plasma verwendet werden,
   - aufzeichnen der Ergebnisse.

2. Verfahren nach Anspruch 1, wobei die Wärmerate infolge einer einzelnen Injektion von LPL in die ITC-Zelle mit menschlichem Plasma geändert wird.

3. Verfahren nach Anspruch 1, wobei die Wärmerate infolge aufeinanderfolgender Injektionen von LPL in die ITC-Zelle mit menschlichem Plasma geändert wird.

4. Verfahren nach Anspruch 1, wobei die Wärmerate proportional zur Reaktionsrate geändert wird.

5. Verfahren nach Anspruch 1, wobei die Wärmerate infolge der Injektion von LPL in eine ITC-Zelle mit 1 ml menschlichem Plasma verändert wird.

6. Verfahren nach Anspruch 1, wobei die Wärmerate infolge der Injektion von LPL in eine ITC-Zelle mit einer menschlichen Plasmasubstratmischung, die menschliches VLDL enthält, verändert wird.

7. Verfahren nach Anspruch 1, wobei die Wärmerate infolge der Injektion von LPL in die ITC-Zelle mit einer menschlichen Plasmasubstratmischung, die eine synthetische Triglyceridemulsion enthält, verändert wird.

8. Verfahren nach Anspruch 1, wobei die Konzentration von LPL im Plasma 22 nM beträgt.

9. Verfahren nach Anspruch 1, wobei die Konzentration von Triglyceriden im Plasma 2,7 mM beträgt.

10. Verfahren nach Anspruch 1, wobei die LPL-Konzentration in der ITC-Zelle durch jede Injektion um 50 pM erhöht wird.

11. Verfahren nach Anspruch 1, wobei die vollständige Hydrolyse von Plasmalipiden durch LPL als Ergebnis aufgezeichnet wird.

12. Verfahren nach Anspruch 1, wobei Unterschiede in der Zugänglichkeit der Lipide für LPL als Ergebnis aufgezeichnet werden.

13. Verfahren nach Anspruch 1, wobei aufgezeichnet wird, wie LPL die Hydrolyse von Lipiden in Plasmaproben katalysieren kann.

14. Verfahren nach Anspruch 1, wobei der Einfluss von zugesetzten Regulatoren auf die LPL-Reaktion im Plasma als Ergebnis aufgezeichnet wird.

**Revendications**

1. Procédé de détermination calorimétrique de l'activité de la lipoprotéine lipase (LPL) dans le plasma humain, comprenant les étapes suivantes:

   - préparation d'au moins un réactif contenant de la LPL par purification de la LPL bovine à partir de lait et dialysage de celle-ci dans une solution tampon TRIS contenant 4 mM de désoxycholate de sodium et ayant un pH de 8,5 à 4 °C,

- introduction d'un échantillon de plasma obtenu à partir d'un être humain,

- préparer l'échantillon de plasma humain à partir d'échantillons de sang en éliminant les cellules par centrifugation pendant 30 minutes à 2000 x g à 4 °C,

- remplir la cellule de calorimétrie à titrage isotherme avec l'échantillon de plasma humain préparé,

- injecter le réactif contenant de la LPL dans la cellule ITC à partir de la seringue-agitateur,

- modifier le taux de chaleur à la suite de l'injection de LPL dans la cellule de calorimétrie à titrage isotherme (ITC) avec du plasma humain;

- augmenter la concentration de LPL dans la cellule ITC par au moins une injection;

- effectuer des mesures de calorimétrie à titrage isotherme, dans lesquelles les mesures ITC sont utilisées pour mesurer l'activité de la LPL dans le plasma humain,

- enregistrer les résultats.

2. Procédé selon la revendication 1, dans lequel le taux de chaleur est modifié à la suite d'une injection unique de LPL dans la cellule ITC avec du plasma humain.

3. Procédé selon la revendication 1, dans lequel le taux de chaleur est modifié à la suite d'injections séquentielles de LPL dans la cellule ITC avec du plasma humain.

4. Procédé selon la revendication 1, dans lequel le taux de chaleur est modifié proportionnellement au taux de réaction.

5. Procédé selon la revendication 1, dans lequel le taux de chaleur est modifié à la suite de l'injection de LPL dans une cellule ITC avec 1 ml de plasma humain.

6. Procédé selon la revendication 1, dans lequel le taux de chaleur est modifié à la suite de l'injection de LPL dans une cellule ITC avec un mélange de substrats de plasma humain contenant du VLDL humain.

7. Procédé selon la revendication 1, dans lequel le taux de chaleur est modifié à la suite de l'injection de LPL dans une cellule ITC avec un mélange de substrats plasmatiques humains contenant une émulsion de triglycérides synthétiques.

8. Procédé selon la revendication 1, dans lequel la concentration de LPL dans le plasma est de 22 nM.

9. Procédé selon la revendication 1, dans lequel la concentration de triglycérides dans le plasma est de 2,7 mM.

10. Procédé selon la revendication 1, dans lequel la concentration en LPL est augmentée dans la cellule ITC de 50 pM à chaque injection.

11. Procédé selon la revendication 1, dans lequel l'hydrolyse totale des lipides plasmatiques par la LPL est enregistrée comme résultat.

12. Procédé selon la revendication 1, dans lequel les différences dans la capacité de la LPL à accéder aux lipides sont enregistrées comme résultat.

13. Procédé selon la revendication 1, dans lequel la capacité de la LPL à catalyser l'hydrolyse des lipides dans les échantillons de plasma est enregistrée comme résultat.

14. Procédé selon la revendication 1, dans lequel l'influence des régulateurs ajoutés sur la réaction de la LPL dans le plasma est enregistrée comme résultat.

FIG 1a

FIG 1b

FIG 1c

FIG 1d

FIG 2

FIG 3a

FIG 3b

FIG 4b

Heat effect (µJ)

NEFA (mM)

FIG 4a

Heat rate (µJ/s)

Time (s)

FIG 4c

FIG 5a

FIG 5b

FIG 6a

FIG 6b

FIG 6c

FIG 6d

FIG 7a

FIG 7b

FIG 7c

FIG 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2012064556 A1, JIN WEIJUN

### Non-patent literature cited in the description

- **WU XULI et al.** Acteoside: A lipase inhibitor from the Chinese tea Ligustrum purpurascenskudingcha. *FOOD CHEMISTRY*, 25 July 2013, vol. 142, 306-3
- **REIMUND MART et al.** Lipoprotein lipase activity and interactions studied in human plasma by isothermal titration calorimetry. *JOURNAL OF LIPID RESEARCH*, 31 December 2016, vol. 58, 279-288
- **ANON**. Triglyceride-mediated pathways and coronary disease: collaborative analysis of 101 studies. *The Lancet*, 2010, vol. 375 (9726), 1634-1639
- **BASU, D. ; MANJUR, J. ; JIN, W.** Determination of lipoprotein lipase activity using a novel fluorescent lipase assay. *Journal of lipid research*, 2011, vol. 52 (4), 826-32
- **BECKSTEAD, J.A. et al.** Structure-function studies of human apolipoprotein A-V: a regulator of plasma lipid homeostasis.. *Biochemistry*, 2003, vol. 42 (31), 9416-23
- **BENGTSSON-OLIVECRONA, G. ; OLIVECRONA, T.** Phospholipase activity of milk lipoprotein lipase.. *Methods in enzymology*, 1991, vol. 197, 345-56
- **BENGTSSON-OLIVECRONA, G. ; SLETTEN, K.** Primary structure of the bovine analogues to human apolipoproteins CII and CIII. Studies on isoforms and evidence for proteolytic processing.. *European journal of biochemistry / FEBS*, 1990, vol. 192 (2), 515-21
- Titration Calorimetry as a Tool to Determine Thermodynamic and Kinetic Parameters of Enzymes.. **BIANCONI, M.L.** Biocalorimetry. John Wiley & Sons, Ltd, 2005, vol. 2, 175-185
- **BRIQUET-LAUGIER, V. ; BEN-ZEEV, O. ; DOOLITTLE, M.H.** Determining lipoprotein lipase and hepatic lipase activity using radiolabeled substrates.. *Methods in molecular biology (Clifton, N.J.)*, 1999, vol. 109, 81-94
- **DALLINGA-THIE, G.M. et al.** Triglyceride-Rich Lipoproteins and Remnants: Targets for Therapy?. *Current cardiology reports*, 2016, vol. 18 (7), 67

- **DIJK, W. ; KERSTEN, S.** Regulation of lipid metabolism by angiopoietin-like proteins.. *Current opinion in lipidology*, 2016, vol. 27 (3), 249-56
- **DO, R. et al.** Exome sequencing identifies rare LDLR and APOA5 alleles conferring risk for myocardial infarction.. *Nature*, 2015, vol. 518 (7537), 102-6
- **DOLE, V.** A relation between non-esterified fatty acids in plasma and the metabolism of glucose.. *The Journal of clinical investigation*, 1956, vol. 35 (2), 150-4
- **EISENBERG, S. ; OLIVECRONA, T.** Very low density lipoprotein. Fate of phospholipids, cholesterol, and apolipoprotein C during lipolysis in vitro.. *Journal of lipid research*, 1979, vol. 20 (5), 614-23
- **ELLIS, R.J.** Macromolecular crowding: obvious but underappreciated.. *Trends in Biochemical Sciences*, 2001, vol. 26 (10), 597-604
- **FREDENRICH, A. et al.** Plasma lipoprotein distribution of apoC-III in normolipidemic and hypertriglyceridemic subjects: comparison of the apoC-III to apoE ratio in different lipoprotein fractions.. *Journal of lipid research*, 1997, vol. 38 (7), 1421-32
- **HAVEL, R.J. ; EDER, H.A. ; BRAGDON, J.H.** The distribution and chemical composition of ultracentrifugally separated lipoproteins in human serum.. *The Journal of clinical investigation*, 1955, vol. 34 (9), 1345-53
- **JOHNSTON, T.P.** Poloxamer 407 as a general lipase inhibitor: its implications in lipid metabolism and atheroma formation in C57BL/6 mice.. *The Journal of pharmacy and pharmacology*, 2010, vol. 62 (12), 1807-12
- **KARPE, F. et al.** Lipoprotein lipase in plasma after an oral fat load: relation to free fatty acids.. *Journal of lipid research*, 1992, vol. 33 (7), 975-84
- **KEI, A.A. et al.** A review of the role of apolipoprotein C-II in lipoprotein metabolism and cardiovascular disease.. *Metabolism: clinical and experimental*, 2012, vol. 61 (7), 906-21

- **KHETARPAL, S.A.** ; **RADER, D.J.** Triglyceride-rich lipoproteins and coronary artery disease risk: new insights from human genetics.. *Arteriosclerosis, thrombosis, and vascular biology*, 2015, vol. 35 (2), e3-9
- **LAFFERTY, M.J. et al.** Angiopoietin-like protein 4 inhibition of lipoprotein lipase: evidence for reversible complex formation.. *The Journal of biological chemistry*, 2013, vol. 288 (40), 28524-34
- **LARSSON, M. et al.** Apolipoproteins C-I and C-III inhibit lipoprotein lipase activity by displacement of the enzyme from lipid droplets.. *The Journal of biological chemistry*, 2013, vol. 288 (47), 33997-4008
- **LOOKENE, A. et al.** Rapid subunit exchange in dimeric lipoprotein lipase and properties of the inactive monomer.. *The Journal of biological chemistry*, 2004, vol. 279 (48), 49964-72
- **LOOKENE, A.** ; **BENGTSSON-OLIVECRONA, G.** Chymotryptic cleavage of lipoprotein lipase. Identification of cleavage sites and functional studies of the truncated molecule.. *European journal of biochemistry / FEBS*, 1993, vol. 213 (1), 185-94
- **LOOKENE, A.** ; **SAVONEN, R.** ; **OLIVECRONA, G.** Interaction of lipoproteins with heparan sulfate proteoglycans and with lipoprotein lipase. Studies by surface plasmon resonance technique.. *Biochemistry*, 1997, vol. 36 (17), 5267-75
- **LOOKENE, A.** ; **SKOTTOVA, N.** ; **OLIVECRONA, G.** *Interactions of lipoprotein lipase with the active-site inhibitor tetrahydrolipstatin (Orlistat) R.,* 1994, vol. 403, 395-403
- **MUSUNURU, K.** ; **KATHIRESAN, S.** Surprises From Genetic Analyses of Lipid Risk Factors for Atherosclerosis.. *Circulation research*, 2016, vol. 118 (4), 579-85
- **NILSSON, S.K. et al.** Apolipoprotein A-V; a potent triglyceride reducer.. *Atherosclerosis*, 2011, vol. 219 (1), 15-21
- **NORDESTGAARD, B.G.** Triglyceride-Rich Lipoproteins and Atherosclerotic Cardiovascular Disease: New Insights From Epidemiology, Genetics, and Biology. *Circulation research*, 2016, vol. 118 (4), 547-63
- **O'BRIEN, P.J. et al.** The novel apolipoprotein A5 is present in human serum, is associated with VLDL, HDL, and chylomicrons, and circulates at very low concentrations compared with other apolipoproteins.. *Clinical chemistry*, 2005, vol. 51 (2), 351-9
- **OLIVECRONA, G.** Role of lipoprotein lipase in lipid metabolism.. *Current opinion in lipidology*, 2016, vol. 27 (3), 233-41
- **OLIVECRONA, G.** ; **BEISIEGEL, U.** Lipid binding of apolipoprotein CII is required for stimulation of lipoprotein lipase activity against apolipoprotein CII-deficient chylomicrons.. *Arteriosclerosis, thrombosis, and vascular biology*, 1997, vol. 17 (8), 1545-9
- Determination and Clinical Significance of Lipoprotein Lipase and Hepatic Lipase. **OLIVECRONA, T.** ; **OLIVECRONA, G.** Handbook of Lipoprotein Testing. AACC Press, 2000, 479-498
- The Ins and Outs of Adipose Tissue. **OLIVECRONA, T.** ; **OLIVECRONA, G.** Cellular Lipid Metabolism. Springer, 2009, 315-369
- **PANTEGHINI, M.** ; **BONORA, R.** ; **PAGANI, F.** Measurement of pancreatic lipase activity in serum by a kinetic colorimetric assay using a new chromogenic substrate.. *Annals of clinical biochemistry*, 2001, vol. 38 (4), 365-70
- **PENNACCHIO, L.A. et al.** An apolipoprotein influencing triglycerides in humans and mice revealed by comparative sequencing. *Science (New York, N.Y.)*, 2001, vol. 294 (5540), 169-73
- **PERRET, B. et al.** Hepatic lipase: structure/function relationship, synthesis, and regulation. *Journal of lipid research*, 2002, vol. 43 (8), 1163-9
- **PETERSON, J. et al.** Fatty acid control of lipoprotein lipase: a link between energy metabolism and lipid transport.. *Proceedings of the National Academy of Sciences of the United States of America*, 1990, vol. 87 (3), 909-13
- **QUINN, D.** ; **SHIRAI, K.** ; **JACKSON, R.L.** Lipoprotein lipase: mechanism of action and role in lipoprotein metabolism.. *Progress in lipid research*, 1983, vol. 22 (1), 35-78
- **RADER, D.J.** New Therapeutic Approaches to the Treatment of Dyslipidemia.. *Cell Metabolism*, 2016, vol. 23 (3), 405-412
- **REIMUND, M. et al.** Evidence for Two Distinct Binding Sites for Lipoprotein Lipase on Glycosylphosphatidylinositol-anchored High Density Lipoprotein-binding Protein 1 (GPIHBP1).. *The Journal of biological chemistry*, 2015, vol. 290 (22), 13919-34
- **ROBAL, T. et al.** Fatty acids bind tightly to the N-terminal domain of angiopoietin-like protein 4 and modulate its interaction with lipoprotein lipase.. *Journal of Biological Chemistry*, 2012, vol. 287 (35), 29739-29752
- **ROBCIUC, M.R. et al.** Quantitation of serum angiopoietin-like proteins 3 and 4 in a Finnish population sample.. *Journal of lipid research*, 2010, vol. 51 (4), 824-31
- **SHAN, L. et al.** The angiopoietin-like proteins ANGPTL3 and ANGPTL4 inhibit lipoprotein lipase activity through distinct mechanisms.. *The Journal of biological chemistry*, 2009, vol. 284 (3), 1419-24
- **SHEN, Y. et al.** *Site-directed Mutagenesis of Apolipoprotein CII to Probe the Role of Its Secondary Structure for Activation of.*, 2010, vol. 285 (10), 7484-7492

- **SONNENBURG, W.K. et al.** GPIHBP1 stabilizes lipoprotein lipase and prevents its inhibition by angiopoietin-like 3 and angiopoietin-like 4. *Journal of lipid research*, 2009, vol. 50 (12), 2421-2429
- **SUKONINA, V. et al.** Angiopoietin-like protein 4 converts lipoprotein lipase to inactive monomers and modulates lipase activity in adipose tissue.. *Proceedings of the National Academy of Sciences of the United States of America*, 2006, vol. 103 (46), 17450-5

- **TORNVALL, P. et al.** Lipoprotein lipase mass and activity in plasma and their increase after heparin are separate parameters with different relations to plasma lipoproteins.. *Arteriosclerosis, thrombosis, and vascular biology*, 1995, vol. 15 (8), 1086-93